# EUROPEAN PATENT APPLICATION

(11) **EP 4 834 333 A1**
(43) Date of publication of application: **04.11.2026**
(21) Application number: 24912452.0
(22) Date of filing: 13.12.2024
(51) Int. Cl.: A61K 9/48, A61K 47/10, A61K 47/26, A61K 47/36, A61K 47/42

(54) **METHOD FOR STABLY PRODUCING SEAMLESS CAPSULES**

(30) Priority: 27.12.2023 JP 2023220887
(71) Applicant: Morishita Jintan Co., Ltd., Osaka-shi, Osaka 540-8566 (JP)
(72) Inventor: YOSHIKADO, Masatomo, Hirakata-shi, Osaka 573-0128 (JP); NISHIKAWA, Takehiro, Hirakata-shi, Osaka 573-0128 (JP); NAKANO, Osami, Hirakata-shi, Osaka 573-0128 (JP); KUWAHARA, Katsumasa, Hirakata-shi, Osaka 573-0128 (JP)
(74) Representative: dompatent
(86) International application number: PCT/JP2024/044246
(87) International publication number: WO 2025/142563

(57) **Abstract**

Provided is a method by which a seamless capsule whose shell hardly cracks can be stably produced when the seamless capsule is produced by the dropping method. The present invention provides: a method for producing a seamless capsule, comprising:
using a capsule production device comprising: a concentric multiple nozzle and a forming tube through which capsule droplets discharged from a discharge port of the concentric multiple nozzle are solidified in a flowing solidifying liquid; the concentric multiple nozzle comprising, provided concentrically from the inner side, a content nozzle and a shell nozzle, and if necessary, one or more intermediate nozzles present between the content nozzle and the shell nozzle,
wherein two kinds of capsule particles, a main capsule particle and a sub capsule particle, are formed in the forming tube;
a particle diameter of the main capsule particle is larger than a particle diameter of the sub capsule particle; and
when a generation ratio of the main capsule particle is 1, a generation ratio of the sub capsule particle is more than 0.5.

## Description

### TECHNICAL FIELD

The present invention relates to a novel method for stably producing a seamless capsule.

### BACKGROUND ART

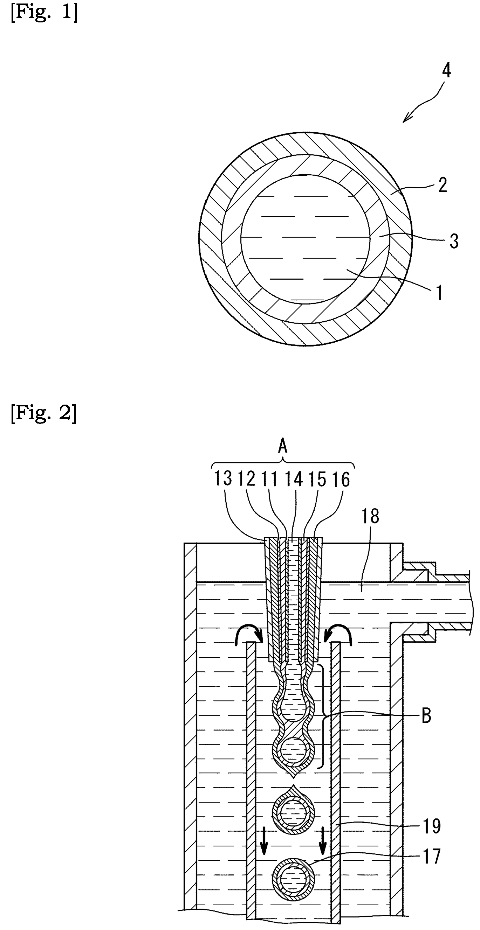

Seamless capsules are usually formed by dropping droplets into a solidifying liquid, which is called dropping method. For example, a seamless capsule 4 illustrated in Fig. 1, constituted by three layers of content 1, an intermediate layer 3, and a shell 2, is produced by using a production device schematically illustrated in Fig. 2. In Fig. 2, a concentric multiple nozzle A has a content nozzle 11, an intermediate layer nozzle 12, and a shell nozzle 13 concentrically formed therein, a content liquid 14, an intermediate layer liquid 15, and a shell liquid 16 are discharged from the lower end (discharge port) of the concentric multiple nozzle A where a solidifying liquid 18 is flowing downward in a forming tube 19, droplets are separated in a forming part B and solidified, and then a seamless capsule 17 is finally formed.

JP-A-2022-26425 (Patent Document 1) discloses a method for producing a seamless capsule that is produced as described above, in which the capsule content (content 1) is blended with a lecithin containing phosphatidylcholine in an amount of 85 wt% or more so that the shell (shell 2) has no defects called "uneven thickness" and "eyes". Here, Patent Document 1 states that both "uneven thickness" and "eyes" cause reduction of shell strength.

However, it is not easy to stably produce a seamless capsule whose shell hardly cracks only by blending the content 1 with a lecithin containing phosphatidylcholine in an amount of 85 wt% or more, as described in Patent Document 1.

### PRIOR ART DOCUMENT

### PATENT DOCUMENT

Patent Document 1: JP-A-2022-26425

### SUMMARY OF THE INVENTION

### PROBLEMS TO BE SOLVED BY THE INVENTION

Therefore, the present invention has been made in view of the above, and an object of the present invention is to provide a method by which a seamless capsule whose shell hardly cracks can be stably produced when the seamless capsule is produced by the dropping method.

### SOLUTIONS TO THE PROBLEMS

The present inventors focused on the fact that when a seamless capsule is produced by the dropping method, a seamless capsule having a small particle diameter is formed in the vicinity of a seamless capsule having a large particle diameter; and found that the two kinds (those having a large particle diameter and those having a small particle diameter) can be actively formed to stably obtain a target seamless capsule (usually, the larger seamless capsule).

That is, the present invention provides the following embodiments:
[1] A method for producing a seamless capsule, comprising:
   using a capsule production device comprising: a concentric multiple nozzle and a forming tube through which capsule droplets discharged from a discharge port of the concentric multiple nozzle are solidified in a flowing solidifying liquid; the concentric multiple nozzle comprising, provided concentrically from the inner side, a content nozzle and a shell nozzle, and if necessary, one or more intermediate nozzles present between the content nozzle and the shell nozzle,
   wherein two kinds of capsule particles, a main capsule particle and a sub capsule particle, are formed in the forming tube;
   a particle diameter of the main capsule particle is larger than a particle diameter of the sub capsule particle; and
   when a generation ratio of the main capsule particle is 1, a generation ratio of the sub capsule particle is more than 0.5.
[2] A method for preventing cracking of a shell of a seamless capsule, comprising:
   using a capsule production device, to produce the seamless capsule, comprising: a concentric multiple nozzle and a forming tube through which capsule droplets discharged from a discharge port of the concentric multiple nozzle are solidified in a flowing solidifying liquid; the concentric multiple nozzle comprising, provided concentrically from the inner side, a content nozzle and a shell nozzle, and if necessary, one or more intermediate nozzles present between the content nozzle and the shell nozzle,
   wherein the droplets discharged from the concentric multiple nozzle are solidified in the forming tube and thereby two kinds of capsule particles, a main capsule particle and a sub capsule particle, are formed;
   a particle diameter of the main capsule particle is larger than a particle diameter of the sub capsule particle; and
   when a generation ratio of the main capsule particle is 1, a generation ratio of the sub capsule particle is more than 0.5.
[3] A method for simultaneously producing two kinds of seamless capsules, comprising:
   using a capsule production device, to produce the seamless capsules, comprising: a concentric multiple nozzle and a forming tube through which capsule droplets discharged from a discharge port of the concentric multiple nozzle are solidified in a flowing solidifying liquid; the concentric multiple nozzle comprising, provided concentrically from the inner side, a content nozzle and a shell nozzle, and if necessary, one or more intermediate nozzles present between the content nozzle and the shell nozzle,
   wherein the droplets discharged from the concentric multiple nozzle are solidified in the forming tube and thereby two kinds of capsule particles, a main capsule particle and a sub capsule particle, are formed;
      a particle diameter of the main capsule particle is larger than a particle diameter of the sub capsule particle; and
      when a generation ratio of the main capsule particle is 1, a generation ratio of the sub capsule particle is more than 0.5.

### EFFECTS OF THE INVENTION

According to the present invention, it is possible to stably produce a seamless capsule whose shell hardly cracks.

### BRIEF DESCRIPTION OF THE DRAWINGS

[Fig. 1] Fig. 1 is a schematic diagram of a three-layer seamless capsule.
[Fig. 2] Fig. 2 is a schematic diagram of a production device of a three-layer seamless capsule through the dropping method.
[Fig. 3] Fig. 3 is a diagram schematically illustrating a pair of a main capsule particle and a sub capsule particle in a solidifying liquid flowing in a forming tube.

### DETAILED DESCRIPTION

The present inventors focused on the fact that two kinds of capsule particles (that is, a main capsule particle and a sub capsule particle) are formed when a seamless capsule is produced by the dropping method. Such two kinds of capsule particles are different in size from each other. According to the basic idea of the present inventors, one capsule particle (specifically, the main capsule particle) is a capsule particle to be produced, and the other capsule particle (specifically, the sub capsule particle), secondarily generated, is a capsule particle basically not to be produced.

Generation of the sub capsule particle is usually considered as a factor for reducing the yield of the main capsule particle. However, the present inventors intensively studied and found that the main capsule particle can be rather stably produced by actively producing the sub capsule particle. Here, the term "stably" refers to the quality stability of the product (seamless capsule) where the shell hardly cracks and the variation of the particle diameter is reduced.

Hereinafter, embodiments of the present invention will be described in detail.

In the method for producing a seamless capsule according to the present invention, the dropping method is employed, and both the main capsule particle and the sub capsule particle are produced. In the production method, when a discharge liquid (jet) is discharged from a nozzle tip in a state where the nozzle tip is present at the liquid surface of a solidifying liquid or in the liquid thereof, the discharge liquid first drops into the solidifying liquid, having a cylindrical shape, and then, through the action of surface tension, the discharge liquid becomes a spherical droplet for forming a capsule, which ideally becomes the target seamless capsule. However, when the discharge liquid having a cylindrical shape becomes a spherical droplet, for example, there is a case where a state like a plurality of particles connected to each other is temporarily made before a droplet particle is formed, and immediately before this finally becomes a spherical droplet, a small particle is formed from the part connecting adjacent particles and extending therebetween, which is considered the sub capsule particle having a small particle diameter.

The sub capsule particle is a particle basically not to be produced. However, as described above, when also the sub capsule particle is actively produced, the properties of the main capsule particle can be stably maintained. That is, when both of the main capsule particle and the sub capsule particle are produced, the main capsule particle hardly cracks, and the variation of the particle diameter of the main capsule particle can be reduced (not generating an irregularly-shaped capsule particle).

The capsule particle to be produced is the main capsule particle in the above description. However, since the present invention is to actively produce the sub capsule particle, the sub capsule particle can be also used as a commercial product as long as there is commercially no problem in quality such as particle diameter, and therefore it is possible that both the main capsule particle and the sub capsule particle are to be produced. Note that, although the main capsule particle naturally has a particle form of a seamless capsule including a shell and content, also the sub capsule particle often has a particle form of a seamless capsule.

It is not required that the sub capsule particle is one kind. The sub capsule particle is not limited as long as it is smaller than the particle diameter of the main capsule particle, and two or more kinds of sub capsule particles smaller than the particle diameter of the main capsule particle may be obtained. However, when the main capsule particle is more stably produced, the variation of the particle diameter of the sub capsule particle is preferably reduced to some extent. Regarding the particle diameter of the sub capsule particle, when the particle diameter of the main capsule particle is 1, the particle diameter of the sub capsule particle is 0.9 or less, and preferably 0.5 or less. The lower limit of the particle diameter of the sub capsule particle is not particularly limited, and for example, the particle diameter of the sub capsule particle may be larger than 0.03. Regarding the sub capsule particle, it is unlikely that the particle diameter of the sub capsule particle is larger than the particle diameter of the main capsule particle, otherwise it would not be clear which is the main capsule particle. Here, the particle diameter is the outer diameter of the capsule particle, and can be measured using a caliper, for example.

Regarding the generation ratio of the sub capsule particle, when the generation ratio of the main capsule particle is 1, the generation ratio of the sub capsule particle is more than 0.5. When the generation ratio of the sub capsule particle is 0.5 or less, the quality of the main capsule particle is hardly stabilized. When the generation ratio of the main capsule particle is 1, the generation ratio of the sub capsule particle is preferably 0.6 or more. The upper limit of the generation ratio of the sub capsule particle may be 1.2 or less, for example. The above-mentioned generation ratio is calculated from the generation number of the main capsule particle and the sub capsule particle. As the method for measuring each generation number, Examples described later employ the number per unit time of each of the main capsule particle and the sub capsule particle passing through a predetermined observation position in the forming tube, but the collected number per unit time of each of the main capsule particle and the sub capsule particle flowing out in the downstream of the forming tube may be employed.

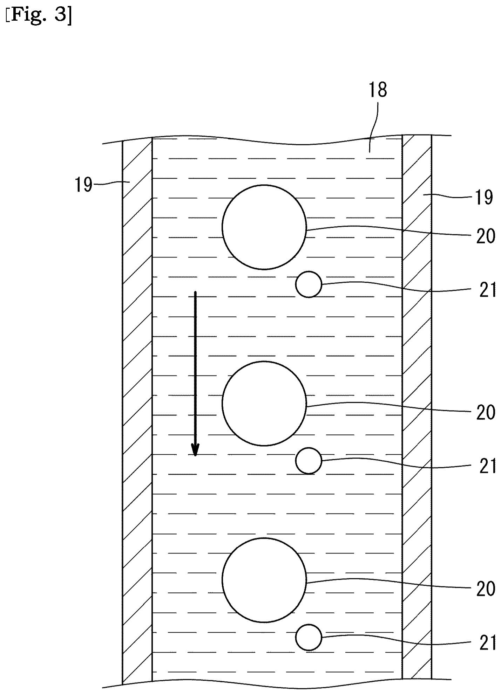

The main capsule particle and the sub capsule particle are usually formed in pairs. For example, the sub capsule particle is often present around the main capsule particle as described in Fig. 3. To represent the state, the sub capsule particle is also referred to as "satellite".

In the production method, in order to actively form the sub capsule particle and produce the main capsule particle with stable quality, the average speed (V) of the solidifying liquid in the forming tube through which the discharge liquid that becomes a capsule particle during production passes, the particle diameter (R) of the main capsule particle, and the generation number (N) of the main capsule particle preferably have the relationship shown in Formula 1 below: $0.7 \leq V / \left(R\times N\right) \leq 2.1$ (Formula 1).

When the relational formula is satisfied, the main capsule particle is stably produced easily. The lower limit of the above Formula 1 is preferably 0.75, more preferably 0.8, and still more preferably 0.85. The upper limit is preferably 2.05, more preferably 2.0, still more preferably 1.95, and still more preferably 1.9. The average speed (V) of the solidifying liquid has a unit of mm/sec, the particle diameter R has a unit of mm, and the generation number N has a unit of number/ sec.

In the above Formula 1, "V/(R × N)" indicates the percentage of the movement distance of the solidifying liquid against the total of the particle diameters of the main capsule particles that pass in the forming tube per unit time. Therefore, as "V/(R × N)" is smaller, the interval between adjacent main capsule particles is smaller, and the particle diameter of the sub capsule particle tends to be smaller, so that the sub capsule particle may hardly be formed. On the other hand, as "V/(R × N)" is larger, the interval between adjacent main capsule particles is larger, and the particle diameter of the sub capsule particle tends to be larger.

### <Capsule particle (or also referred to as "seamless capsule")>

The capsule particle usually has a shell (or outermost layer) and content (content encapsulated in the shell or inner layer). In other words, the capsule particle has content and a shell covering the content. In the present invention, the capsule particle may have an intermediate layer between the shell and the content. The intermediate layer is provided to protect the content and the shell from each other, and is provided to prevent deterioration of the performance of the content. Note that, in the present specification, the term "capsule particle" means both "main capsule particle" and "sub capsule particle".

### <Content>

In the capsule particle, the state of the content is not particularly limited, and the content may be liquid, solid, or semi-solid at room temperature, for example. Examples of the component that can be blended in the content include fatty acid (such as caprylic acid, capric acid, eicosapentaenoic acid, docosahexaenoic acid, oleic acid, and linoleic acid), fatty acid ester [such as triglyceride (glycerin ester (fat) such as medium-chain fatty acid triglyceride (such as MCT); and non-glycerin fatty acid ester such as isopropyl myristate)], vegetable oil (for example, olive oil, sunflower oil, corn oil, peanut oil, grape seed oil, wheat germ oil, rapeseed oil, jojoba oil, safflower oil), hardened oil, mineral oil, silicone oil, sucrose fatty acid ester, liquid paraffin, squalane, perfume (such as ester, alcohol, aldehyde, ketone, phenol, ether, lactone, hydrocarbon, nitrogen-containing compound, sulfur-containing compound, and acid), and functional component (such as vitamin A, vitamin D, vitamin E, vitamin B1, vitamin B2, vitamin B6, vitamin B12, vitamin C, folic acid, niacin, pantothenic acid, biotin, caffeine, iron, copper, zinc, manganese, selenium, chromium, molybdenum, polyglutamic acid, Ampelopsis cantoniensis polyphenol, blueberry extract, black currant extract, bilberry extract, Salacia extract, ginseng powder, gokahi, licorice, peony, cinnamon, fennel, amomum seed, Momordica grosvenori, Bifidobacterium, lactic bacteria, and yeast).

The functional component means a component expected to exert a physiological action on a living body when present in the living body. The functional component is not limited to the above, and any component can be used according to a physiological action on a living body.

The perfume is not particularly limited as long as it can impart perfume to the content, and any of natural perfume and synthetic perfume may be used. The perfume may be used singly or in combination of two or more kinds thereof. The natural perfume is not particularly limited, and examples thereof include oils such as rose, jasmine, bitter orange, neroli, chamomile, ylang-ylang, geranium, eucalyptus, tea tree, petitgrain, satsuma mandarin, orange, lemon, lime, bergamot, pepper, juniper, vanilla, sandalwood, pine, hinoki, cinnamon, frankincense, myrrh, vetiver, spikenard, orris root, lavender, lemongrass, basil, rosemary, mint (such as spearmint, menthol, and peppermint), and berry (such as blueberry, cranberry, lingonberry, huckleberry, raspberry, blackberry, loganberry, salmonberry, boysenberry, strawberry, bilberry, elderberry, and gooseberry). The synthetic perfume is not particularly limited as long as it is conventionally used for the purpose of imparting fragrance and flavor.

When the content is solid or semi-solid at room temperature, the content may contain a lipid (specifically,fats and oils) having a rising melting point of 24°C to 55°C. The "rising melting point" is defined as "temperature at which a sample sufficiently softens and floats in an open capillary tube" and is measured in accordance with ISO6321:2002.

The fats and oils are glycerin esters of fatty acids, and may be any of monoglycerides, diglycerides, and triglycerides. The fatty acid in the fats and oils preferably contains 10 to 20 carbon atoms. Such a fatty acid is, for example, at least one selected from the group consisting of lauric acid (12 carbon atoms), myristic acid (14 carbon atoms), palmitic acid (16 carbon atoms), stearic acid (18 carbon atoms), oleic acid (18 carbon atoms: 1 unsaturated double bond), linoleic acid (18 carbon atoms: 2 unsaturated double bonds), linolenic acid (18 carbon atoms: 3 unsaturated double bonds), arachidic acid (20 carbon atoms), and eicosenoic acid (20 carbon atoms: 1 unsaturated double bond), but is not limited to the above, and any fatty acid can be used.

When the content is solid or semi-solid at room temperature, the content may contain fats and oils having a rising melting point of 60°C or higher. For convenience of explanation, the fats and oils having a rising melting point of 60°C or higher are referred to as second fats and oils, and the fats and oils having a rising melting point of 24 to 55°C are referred to as first fats and oils, in some cases. The rising melting point of the second fats and oils is usually 190°C or lower, preferably 150°C or lower, more preferably 100°C or lower, and still more preferably 80°C or lower.

The second fats and oils are fatty acid glycerin esters similarly to the first fats and oils, and may be at least one selected from the group consisting of a glycerin fatty acid ester other than the fats and oils used for the first fats and oils, a polyglycerin fatty acid ester, and a beeswax. The polyglycerin fatty acid ester is an esterified product between a fatty acid and a polyglycerin formed by condensation of a plurality of glycerin molecules, and the number of glycerin skeletons constituting the polyglycerin, the number of fatty acid molecules, and the kind of fatty acid are not particularly limited as long as the second fats and oils have a rising melting point of 60°C or higher. The fatty acid contained in the fatty acid ester or the polyglycerin fatty acid ester preferably has 10 to 30 carbon atoms. Such a fatty acid is, for example, at least one selected from the group consisting of lauric acid (12 carbon atoms), myristic acid (14 carbon atoms), palmitic acid (16 carbon atoms), palmitoleic acid (16 carbon atoms: 1 unsaturated double bond), stearic acid (18 carbon atoms), oleic acid (18 carbon atoms: 1 unsaturated double bond), linoleic acid (18 carbon atoms: 2 unsaturated double bonds), linolenic acid (18 carbon atoms: 3 unsaturated double bonds), arachidic acid (20 carbon atoms), eicosenoic acid (20 carbon atoms: 1 unsaturated double bond), behenic acid (22 carbon atoms), and lignoceric acid (24 carbon atoms), but is not limited to the above, and any fatty acid can be used.

In the capsule particle of the present invention, the content may be further appropriately blended with an excipient, a stabilizer, a surfactant, an auxiliary agent, a foaming agent, or the like. The amount of these blending agents is not particularly limited as long as the effects of the present invention are not impaired.

### <Shell>

In the capsule particle of the present invention, the outside of the content is covered with a shell. The shell contains a water-soluble natural polymer. The water-soluble natural polymer is selected, for example, from gelatin, casein, zein, pectin or derivatives thereof, alginic acid or salts thereof, agar, gellan gum, carrageenan, furcellaran, chitosan, curdlan, starch, modified starch, pullulan, mannan, and mixtures thereof. Of course, the water-soluble natural polymer is not limited thereto. These water-soluble natural polymers are preferably in a range of 50 wt% to 90 wt% based on the total solid content weight of the shell composition of the seamless capsule. When alginate, gellan gum, pectin, or carrageenan is used, an alkali metal salt, an alkaline earth metal salt, an ammonium salt, or the like may be appropriately added.

In the capsule particle of the present invention, the shell may further contain a plasticizer in order to impart flexibility in a dry state. Examples of the plasticizer include glycerin and sorbitol. The blending amount of the plasticizer is 1 to 50 wt%, preferably 5 to 40 wt%, and more preferably 15 to 35 wt% based on the total weight of the dried shell. When the blending amount of the plasticizer is less than 1 wt%, the shell cannot withstand vacuum drying or cannot maintain sufficient flexibility in a dry state to result in cracking. When the blending amount of the plasticizer is more than 50 wt%, the shell is softened to adhere and melt at a high temperature.

In the capsule particle of the present invention, the shell may contain various additives usually used in this field, such as perfume, sweetener, colorant, and preservative such as paraben, in addition to the above composition, as necessary. When such an additive is used, the total content of all the additives is, for example, 0.01 to 10 wt%, and preferably 0.1 to 5 wt% based on the total solid content weight in the composition of the shell of the capsule.

The thickness (shell thickness) of the shell is not particularly limited since it can be arbitrarily selected according to the intended use of the capsule particle. For example, the shell thickness may be 10 to 600 µm, may be 30 to 400 µm, or may be 40 to 250 µm.

### <Intermediate layer>

The capsule particle of the present invention further includes an intermediate layer as necessary. The intermediate layer is formed when the content has multiple layers or the shell layer has multiple layers. For example, when the content is liquids having different viscosities, the liquid having a lower viscosity is used for the inner layer, and the liquid having a higher viscosity is used for the second layer (the second layer from the inner layer) so that multiple layers can be made. Alternatively, when the content to be used is a plurality of fats and oils having different melting points, the inner layer has the lowest melting point, and the second layer has a higher melting point than the inner layer so that multiple layers can be made. Similarly, for example, the shell may have a shell having low oxygen permeability as the outermost layer, and a usual water-soluble natural polymer shell formed on the inner side thereof as the second layer from the outer side.

### <Method for producing capsule particle>

The method for producing a capsule particle according to the present invention can be performed with a production device as illustrated in Fig. 2. Fig. 2 is a diagram schematically illustrating a production device to be used in the method for producing a capsule particle according to the present invention, and the diagram used for describing Patent Document 1 is used therefor. The production device includes a concentric multiple nozzle A and a cooling tube 19 through which a solidifying liquid flows down.

Fig. 2 will be described. Fig. 2 illustrates a device to produce a three-layer capsule particle. The content liquid 14 is discharged from the tip (discharge port) of a nozzle 11 on the inner side, and the shell liquid 16 is discharged from the tip (discharge port) of a nozzle 13 on the outer side. Fig. 2 illustrates a case where the intermediate layer includes one layer, and therefore, the intermediate layer liquid 14 is discharged from the tip (discharge port) of an intermediate nozzle 12 disposed between the nozzle 11 and the nozzle 13. Each nozzle is concentrically formed based on the central axis of the nozzle 11 on the inner side. Although Fig. 2 includes the nozzle 12 for the intermediate layer to form the intermediate layer, the present invention also encompasses a case where no intermediate layer nozzle is included, or a case where a plurality of intermediate layer nozzles are included. Here, in the present specification, the intermediate layer nozzle and the intermediate nozzle refer to the same configuration.

The arrow present in Fig. 2 indicates the direction in which the solidifying liquid 18 flows in the forming tube 19. The solidifying liquid 18 flows inside the forming tube 19 in the downward direction. Droplets (droplets forming capsule particles) discharged from the concentric multiple nozzle are discharged into the solidifying liquid 18 in the forming tube 19, and initially become a rod-shaped discharge liquid B in which the discharge liquid continues long, and then become spherical through surface tension while the discharge liquid is going down in the forming tube 19, and are solidified at the same time, so that a capsule particle 17 is formed.

In the present invention, as illustrated in Fig. 3, the capsule particle is formed such that the two kinds, a main capsule particle 20 and a sub capsule particle 21, are paired as much as possible. Fig. 3 is a diagram schematically illustrating that the main capsule particle 20 and the sub capsule particle 21 are paired in the solidifying liquid 18 flowing in the arrow direction in the lower part of the forming tube 19. Although Fig. 2 describes only the main capsule particle 17 as the capsule particle, adjustment is performed so that a sub capsule particle is also present in the vicinity of the capsule particle. Note that, Fig. 3 illustrates that the sub capsule particle 21 is paired with the main capsule particle 20, but a state where one main capsule particle 20 has zero or a plurality of sub capsule particles may be partially made in the flow direction of the solidifying liquid 18. However, when the main capsule particle 20 is stably produced, the main capsule particle 20 and the sub capsule particle 21 are preferably formed in pairs, as in Fig. 3.

As described above, the present invention requires that the particle diameter of the main capsule particle is larger than the particle diameter of the sub capsule particle; and when the generation ratio of the main capsule particle is 1, the generation ratio of the sub capsule particle is more than 0.5.

The particle diameter (diameter of the sphere) of the main capsule particle is considered based on the target particle diameter, and therefore is not particularly limited. For example, the diameter is 0.3 to 10 mm, and preferably 1 to 8 mm. When the diameter of the main capsule particle is less than 0.3 mm, production thereof is difficult by the dropping method, which is used in the present invention. Although the main capsule particle having a particle diameter of more than 10 mm is not a particular problem, when the main capsule particle is orally ingested, swallowing thereof tends to be difficult.

There is no problem as long as the particle diameter of the sub capsule particle is smaller than the particle diameter of the main capsule particle. However, when the particle diameter of the sub capsule particle is larger than the particle diameter of the main capsule particle, it is not clear which is the main capsule particle, and control thereof is difficult, which is not preferable.

As described above, in the present invention, in order to stably produce the capsule particle, the average speed (V) of the solidifying liquid in the forming tube, the particle diameter (R) of the main capsule particle, and the generation number (N) of the main capsule particle preferably satisfy Formula 1 below. $0.7 \leq V / \left(R\times N\right) \leq 2.1$ (Formula 1)

Within the range, the main capsule particle can be stably produced. Since the sub capsule particle is actively produced even secondarily, the sub capsule particle can also be produced, and the sub capsule particle is generated stably and steadily, so that the sub capsule particle (particle diameter smaller than that of the main capsule particle) can also be produced.

Regarding the obtained capsule particles, the main capsule particles and the sub capsule particles are separated from each other, and then the target particles are dried. Thereby, the target capsule particle (seamless capsule) can be produced.

When the capsule particle of the present invention is produced, a capsule shell preparation liquid and a capsule content liquid are prepared in advance. Then, each of the capsule shell preparation liquid and the capsule content liquid is discharged from the multiple nozzle to form a composite jet.

The capsule shell preparation liquid contains a natural polymer and a solvent. The natural polymer is a component that solidifies the capsule shell preparation liquid, and is water-soluble. Description of the natural polymer quotes the description of the above-described water-soluble natural polymer.

The solvent is a liquid that disperses the components in the capsule shell preparation liquid. For example, such a solvent is at least one selected from the group consisting of water and ethanol. Examples of the water include tap water, ion-exchanged water, distilled water, ultrapure water, and a mixture thereof.

The capsule shell preparation liquid may further contain a plasticizer. The plasticizer is a component that imparts flexibility to the solidified product of the capsule shell preparation liquid. The plasticizer allows the shell to maintain sufficient flexibility and hardly crack, especially in a dry capsule, which is a dry product of a wet capsule (capsule particle in a wet state). Examples of the plasticizer include polyhydric alcohols such as glycerin and sorbitol. The plasticizer is not limited to the above, and any component can be applied.

The capsule shell preparation liquid may further contain additives such as pigment, taste component, preservative, or perfume, as necessary.

In the method for producing a capsule particle, the solidifying liquid is typically at 20°C or lower, and preferably 1 to 18°C. The temperature of each liquid extruded from each nozzle is not particularly limited, and is typically 15 to 70°C, and preferably 20 to 65°C.

As the solidifying liquid, for example, medium-chain fatty acid triglyceride (MCT), vegetable fats and oils (such as coconut oil, sunflower oil, safflower oil, sesame oil, rapeseed oil, grape seed oil, and mixtures thereof), liquid paraffin, and mixtures thereof can be used.

In the above production method, although not illustrated, a vibration means may be used to apply moderate vibration to the composite jet flow (B in Fig. 2) so that the composite jet is favorably cut.

The capsule particle of the present invention can be subjected to water washing, heat sterilization, and heat disinfection, as necessary, depending on the use conditions.

### [Examples]

The present invention will be described more specifically with reference to the following Examples, but the present invention is not limited thereto. In Examples, "parts" and "%" are based on weight unless otherwise specified.

In Examples and Comparative Examples, the following components were used.
· Medium-chain fatty acid triglyceride (Density: 0.960 g/cm³ (10°C)
· Gelatin: Gelatin CP-771 manufactured by JELLICE Co., Ltd.
· Agar: Agar T-1 manufactured by Ina Food Industry Co., Ltd.

### Examples 1 to 5 and Comparative Examples 1 to 2

Content liquid: The capsule content liquid was prepared from only medium-chain fatty acid triglyceride (MCT).

Capsule shell preparation liquid: 80 parts of gelatin, 20 parts of sorbitol, and 400 parts of ion-exchanged water were mixed until the mixture became uniform, and then the mixture was heated and dissolved at 60°C to prepare a capsule shell preparation liquid. The viscosity of the capsule shell preparation liquid was 50 mPa·s (60°C).

Next, in a seamless capsule production device as exemplified in Fig. 2 (manufactured by Morishita Jintan Co.,Ltd.), a concentric double nozzle was used to discharge the capsule content liquid from the innermost nozzle and the capsule shell preparation liquid from the outermost layer nozzle at the same time into a solidifying liquid at 10°C such that the solid content of the capsule shell preparation liquid was 30 parts based on 70 parts of the capsule content liquid, and thereby a wet capsule having a two-layer structure was formed.

When the capsule was produced, it was confirmed that the main capsule particle and the sub capsule particle were being produced in pairs continuously in the forming tube. At this time, a moving image obtained by photographing the vicinity of the center of the length of the forming tube was used, and the number of each of the main capsule particle and the sub capsule particle passing through the observation position was measured for 10 seconds, so that the generation number of each of the main capsule particle and the sub capsule particle (unit: number/sec) was calculated. The average flow rate of the solidifying liquid was calculated as follows: the weight of the solidifying liquid flowing out for 10 seconds from the forming tube was measured, and the weight of the solidifying liquid was introduced into Formula 2 below. 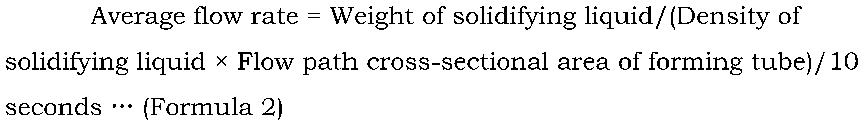 Average flow rate = Weight of solidifying liquid/ (Density of solidifying liquid × Flow path cross-sectional area of forming tube)/ 10 seconds (Formula 2)

In accordance with the evaluation methods below, Formulability I (cracking) and Formulability II (variation in particle diameter) of the main capsule particle were evaluated; and the presence or absence of irregularly-shaped sub capsule particles, the generation rate of irregularly-shaped sub capsule particles, and the variation in the particle diameter of the sub capsule particle were evaluated as described below, and the evaluation results thereof are shown in Table 1. Table 1 describes: the particle diameter (mm) of the main capsule particle; the percentage of the average speed of the solidifying liquid against the product of the particle diameter of the main capsule particle and the generation number of the main capsule particle (V/(R × N)); the particle diameter (mm) of the sub capsule particle; the ratio between the particle diameter of the sub capsule particle and the particle diameter of the main capsule particle; and the generation ratio of the sub capsule particle when the generation ratio of the main capsule particle is 1.

### <Evaluation method>

### [Formulability I (cracking)]

A moving image obtained by photographing the vicinity of the center of the length of the forming tube was used, and 100 main capsule particles passing through the observation position were observed. The proportion of the number of the cracked main capsule particles within the 100 particles was calculated in terms of percentage.

### [Formulability II (variation in particle diameter)]

Twenty main capsule particles were randomly collected. Then, the particle diameter of each main capsule particle was measured with a caliper, and the average particle diameter, standard deviation, and variation coefficient were calculated from the measured values. The percentage of the variation coefficient was used as the evaluation result in "Formulability II".

### [Generation rate of irregularly-shaped sub capsule particles]

One hundred sub capsule particles were randomly collected. Then, the appearance of each sub capsule particle was visually checked, and the number of capsules whose appearance was a shape other than a sphere (irregular shape such as a water droplet shape) was counted. Thereafter, the value obtained when the number of irregularly-shaped particles was divided by 100 particles was calculated in terms of percentage.

### [Variation in particle diameter of sub capsule particle]

Twenty sub capsule particles were randomly collected. Then, the particle diameter of each sub capsule particle was measured with a caliper, and the average particle diameter, standard deviation, and variation coefficient were calculated from the measured values. The percentage of the variation coefficient was used as the evaluation result in "Formulability II".

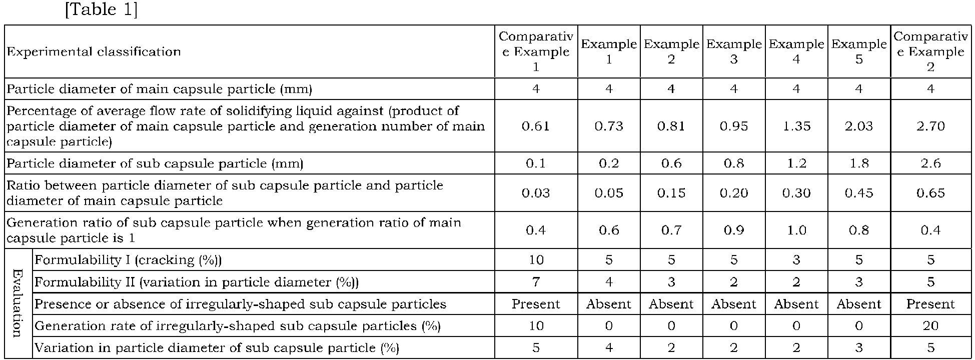

### Examples 6 to 9 and Comparative Examples 3 to 4

Content liquid: The capsule content liquid was prepared from only MCT.

Capsule shell preparation liquid: 68 parts of agar, 32 parts of sodium alginate, and 6000 parts of ion-exchanged water were mixed until the mixture became uniform, and then the mixture was heated and dissolved at a temperature of 105°C in an autoclave to prepare a capsule shell preparation liquid. The viscosity of the capsule shell preparation liquid was 70 mPa·s (80°C).

Next, in a seamless capsule production device as exemplified in Fig. 2 (manufactured by Morishita Jintan Co.,Ltd.), a concentric double nozzle was used to discharge the capsule content liquid from the innermost nozzle and the capsule shell preparation liquid from the outermost layer nozzle at the same time into a solidifying liquid at 10°C such that the solid content of the capsule shell preparation liquid was 1.63 parts based on 100 parts of the capsule content liquid, and thereby a wet capsule having a two-layer structure was formed.

When the capsule was produced, it was confirmed that the main capsule particle and the sub capsule particle were being produced in pairs continuously in the forming tube. At this time, the generation number of each of the main capsule particle and the sub capsule particle and the average flow rate of the solidifying liquid were calculated in the same manner as in Example 1.

In accordance with the evaluation methods above, Formulability I (cracking) and Formulability II (variation in particle diameter) of the main capsule particle were evaluated; and the presence or absence of irregularly-shaped sub capsule particles, the generation rate of irregularly-shaped sub capsule particles, and the variation in the particle diameter of the sub capsule particle were evaluated, and the evaluation results thereof are shown in Table 2. The same items as those in Table 1 are also described in Table 2.

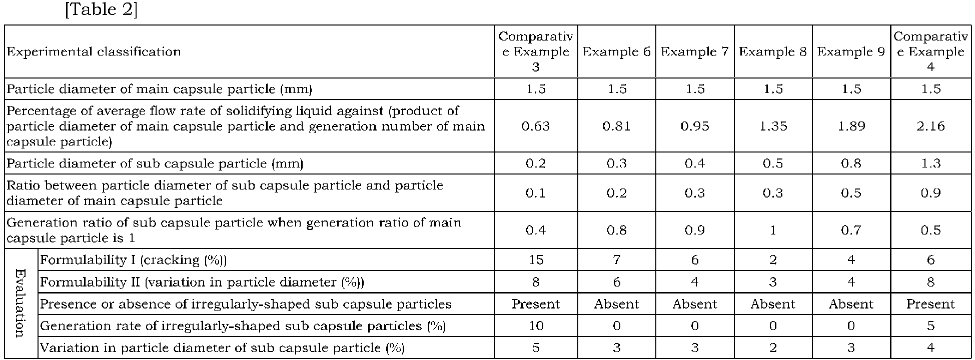

Examples 1 to 5 and Comparative Examples 1 to 2 are examples in which the particle diameter of the main capsule particle is 4 mm, and the sub capsule particle having a particle diameter smaller than the particle diameter of the main capsule particle is generated in all of the examples. However, in Comparative Examples 1 to 2, when the generation ratio of the sub capsule particle is 1, the generation ratio of the sub capsule particle is less than 0.5. In Comparative Examples 1 to 2, the main capsule particle significantly cracks and varies, and the capsule particle is not stably produced. In Examples 1 to 5, the main capsule particle is less cracked, and the variation of the particle diameter is also small. In addition, in all Examples, an extremely small number of irregularly-shaped sub capsules are generated, and the variation of the particle diameter of the sub capsule particle is also small. Note that, in Comparative Example 2, cracking (%) of the main capsule particle is comparable to that in Examples, but the particle diameter of the main capsule particle highly varies, and also a large number of irregularly-shaped sub capsule particles are generated.

Examples 6 to 9 and Comparative Examples 3 to 4 are examples in which the particle diameter of the main capsule particle is 1.5 mm, and the sub capsule particle having a particle diameter smaller than the particle diameter of the main capsule particle is generated in all of the examples. However, in Comparative Examples 3 to 4, when the generation ratio of the sub capsule particle is 1, the generation ratio of the sub capsule particle is less than 0.5. In Comparative Examples 3 to 4, the main capsule particle significantly cracks and varies, and the main capsule particle is not stably produced. In Examples 1 to 5, the main capsule particle is less cracked, and the variation of the particle diameter is also small. In addition, in all Examples, an extremely small number of irregularly-shaped sub capsules are generated, and the variation of the particle diameter of the sub capsule particle is also small. Note that, in Comparative Example 4, cracking (%) of the main capsule particle is comparable to that in Examples, but the particle diameter of the main capsule particle highly varies, and also a large number of irregularly-shaped sub capsule particles are generated.

In Examples, the sub capsule particle is actively produced, so that the particle diameter of the main capsule particle is stabilized, cracking is reduced, and the main capsule particle can be stably produced. In addition, since the particle diameter of the sub capsule particle is also stable, the sub capsule particle can be also produced. In the sub capsule particle, the two layers, the content and the shell, are clearly formed.

### <Additional embodiments of present invention>

[1] A method for producing a seamless capsule, comprising:
   using a capsule production device comprising: a concentric multiple nozzle and a forming tube through which capsule droplets discharged from a discharge port of the concentric multiple nozzle are solidified in a flowing solidifying liquid; the concentric multiple nozzle comprising, provided concentrically from the inner side, a content nozzle and a shell nozzle, and if necessary, one or more intermediate nozzles present between the content nozzle and the shell nozzle,
   wherein two kinds of capsule particles, a main capsule particle and a sub capsule particle, are formed in the forming tube;
   a particle diameter of the main capsule particle is larger than a particle diameter of the sub capsule particle; and
   when a generation ratio of the main capsule particle is 1, a generation ratio of the sub capsule particle is more than 0.5.
[2] The method for producing a seamless capsule according to [1], wherein the main capsule particle, paired with the sub capsule particle, is formed.
[3] The method for producing a seamless capsule according to [1] or [2], wherein an average flow rate of a solidifying liquid in a forming tube for the capsule particles, a particle diameter of the main capsule particle, and a generation number of the main capsule particle satisfy a relationship below: $0.7 \leq V / \left(R\times N\right) \leq 2.1$ (Formula 1) wherein, in Formula 1, V is an average flow rate of a solidifying liquid; R is a particle diameter of a main capsule particle; and N is a generation number of a main capsule particle.
[4] The method for producing a seamless capsule according to any one of [1] to [3], wherein the main capsule particle is separated from the sub capsule particle, and then dried.
[5] A method for preventing cracking of a shell of a seamless capsule, comprising:
   using a capsule production device, to produce the seamless capsule, comprising: a concentric multiple nozzle and a forming tube through which capsule droplets discharged from a discharge port of the concentric multiple nozzle are solidified in a flowing solidifying liquid; the concentric multiple nozzle comprising, provided concentrically from the inner side, a content nozzle and a shell nozzle, and if necessary, one or more intermediate nozzles present between the content nozzle and the shell nozzle,
   wherein the droplets discharged from the concentric multiple nozzle are solidified in the forming tube and thereby two kinds of capsule particles, a main capsule particle and a sub capsule particle, are formed;
   a particle diameter of the main capsule particle is larger than a particle diameter of the sub capsule particle; and
   when a generation ratio of the main capsule particle is 1, a generation ratio of the sub capsule particle is more than 0.5.
[6] The method for preventing cracking of a shell of a seamless capsule according to [5], wherein the main capsule particle, paired with the sub capsule particle, is formed.
[7] The method for preventing cracking of a shell of a seamless capsule according to [5] or [6], wherein an average flow rate of a solidifying liquid in a forming tube for the capsule particles, a particle diameter of the main capsule particle, and a generation number of the main capsule particle satisfy a relationship below: $0.7 \leq V / \left(R\times N\right) \leq 2.1$ (Formula 1) wherein, in Formula 1, V is an average flow rate of a solidifying liquid; R is a particle diameter of a main capsule particle; and N is a generation number of a main capsule particle.
[8] The method for preventing cracking of a shell of a seamless capsule according to any one of [5] to [7], wherein the main capsule particle is separated from the sub capsule particle, and then dried.
[9] A method for simultaneously producing two kinds of seamless capsules, comprising:
   using a capsule production device, to produce the seamless capsules, comprising: a concentric multiple nozzle and a forming tube through which capsule droplets discharged from a discharge port of the concentric multiple nozzle are solidified in a flowing solidifying liquid; the concentric multiple nozzle comprising, provided concentrically from the inner side, a content nozzle and a shell nozzle, and if necessary, one or more intermediate nozzles present between the content nozzle and the shell nozzle,
   wherein the droplets discharged from the concentric multiple nozzle are solidified in the forming tube and thereby two kinds of capsule particles, a main capsule particle and a sub capsule particle, are formed;
   a particle diameter of the main capsule particle is larger than a particle diameter of the sub capsule particle; and
   when a generation ratio of the main capsule particle is 1, a generation ratio of the sub capsule particle is more than 0.5.
[10] The method for simultaneously producing two kinds of seamless capsules according to [9], wherein the main capsule particle, paired with the sub capsule particle, is formed.
[11] The method for simultaneously producing two kinds of seamless capsules according to [9] or [10], wherein an average flow rate of a solidifying liquid in a forming tube for the capsule particles, a particle diameter of the main capsule particle, and a generation number of the main capsule particle satisfy a relationship below: $0.7 \leq V / \left(R\times N\right) \leq 2.1$ (Formula 1) wherein, in Formula 1, V is an average flow rate of a solidifying liquid; R is a particle diameter of a main capsule particle; and N is a generation number of a main capsule particle.
[12] The method for simultaneously producing two kinds of seamless capsules according to any one of [9] to [11], wherein the main capsule particle and the sub capsule particle are separated from each other, and then dried, respectively.

### REFERENCE SIGNS LIST

- 1: Content
- 2: Shell
- 3: Intermediate layer
- 11: Content nozzle
- 12: Intermediate layer nozzle
- 13: Shell nozzle
- 14: Content liquid
- 15: Intermediate layer liquid
- 16: Shell liquid
- 17: Seamless capsule
- 18: Solidifying liquid
- 19: Forming tube
- 20: Main capsule particle
- 21: Sub capsule particle
- A: Concentric multiple nozzle
- B: Forming part

## Claims

1. A method for producing a seamless capsule, comprising:
using a capsule production device comprising: a concentric multiple nozzle and a forming tube through which capsule droplets discharged from a discharge port of the concentric multiple nozzle are solidified in a flowing solidifying liquid; the concentric multiple nozzle comprising, provided concentrically from the inner side, a content nozzle and a shell nozzle, and if necessary, one or more intermediate nozzles present between the content nozzle and the shell nozzle,
wherein two kinds of capsule particles, a main capsule particle and a sub capsule particle, are formed in the forming tube;
a particle diameter of the main capsule particle is larger than a particle diameter of the sub capsule particle; and
when a generation ratio of the main capsule particle is 1, a generation ratio of the sub capsule particle is more than 0.5.

2. The method for producing a seamless capsule according to claim 1, wherein the main capsule particle, paired with the sub capsule particle, is formed.

3. The method for producing a seamless capsule according to claim 1 or 2, wherein an average flow rate of a solidifying liquid in the forming tube for the capsule particles, a particle diameter of the main capsule particle, and a generation number of the main capsule particle satisfy a relationship below: $0.7 \leq V / \left(R\times N\right) \leq 2.1$ (Formula 1) wherein, in Formula 1, V is an average flow rate of the solidifying liquid; R is a particle diameter of the main capsule particle; and N is a generation number of the main capsule particle.

4. The method for producing a seamless capsule according to any one of claims 1 to 3, wherein the main capsule particle is separated from the sub capsule particle, and then dried.

5. A method for preventing cracking of a shell of a seamless capsule, comprising:
using a capsule production device, to produce the seamless capsule, comprising: a concentric multiple nozzle and a forming tube through which capsule droplets discharged from a discharge port of the concentric multiple nozzle are solidified in a flowing solidifying liquid; the concentric multiple nozzle comprising, provided concentrically from the inner side, a content nozzle and a shell nozzle, and if necessary, one or more intermediate nozzles present between the content nozzle and the shell nozzle,
wherein the droplets discharged from the concentric multiple nozzle are solidified in the forming tube and thereby two kinds of capsule particles, a main capsule particle and a sub capsule particle, are formed;
a particle diameter of the main capsule particle is larger than a particle diameter of the sub capsule particle; and
when a generation ratio of the main capsule particle is 1, a generation ratio of the sub capsule particle is more than 0.5.

6. The method for preventing cracking of a shell of a seamless capsule according to claim 5, wherein the main capsule particle, paired with the sub capsule particle, is formed.

7. The method for preventing cracking of a shell of a seamless capsule according to claim 5 or 6, wherein an average flow rate of the solidifying liquid in the forming tube for the capsule particles, a particle diameter of the main capsule particle, and a generation number of the main capsule particle satisfy a relationship below: $0.7 \leq V / \left(R\times N\right) \leq 2.1$ (Formula 1) wherein, in Formula 1, V is an average flow rate of the solidifying liquid; R is a particle diameter of the main capsule particle; and N is a generation number of the main capsule particle.

8. The method for preventing cracking of a shell of a seamless capsule according to any one of claims 5 to 7, wherein the main capsule particle is separated from the sub capsule particle, and then dried.

9. A method for simultaneously producing two kinds of seamless capsules, comprising:
using a capsule production device, to produce the seamless capsules, comprising: a concentric multiple nozzle and a forming tube through which capsule droplets discharged from a discharge port of the concentric multiple nozzle are solidified in a flowing solidifying liquid; the concentric multiple nozzle comprising, provided concentrically from the inner side, a content nozzle and a shell nozzle, and if necessary, one or more intermediate nozzles present between the content nozzle and the shell nozzle,
wherein the droplets discharged from the concentric multiple nozzle are solidified in the forming tube and thereby two kinds of capsule particles, a main capsule particle and a sub capsule particle, are formed;
a particle diameter of the main capsule particle is larger than a particle diameter of the sub capsule particle; and
when a generation ratio of the main capsule particle is 1, a generation ratio of the sub capsule particle is more than 0.5.

10. The method for simultaneously producing two kinds of seamless capsules according to claim 9, wherein the main capsule particle, paired with the sub capsule particle, is formed.

11. The method for simultaneously producing two kinds of seamless capsules according to claim 9 or 10, wherein an average flow rate of the solidifying liquid in the forming tube for the capsule particles, a particle diameter of the main capsule particle, and a generation number of the main capsule particle satisfy a relationship below: $0.7 \leq V / \left(R\times N\right) \leq 2.1$ (Formula 1) wherein, in Formula 1, V is an average flow rate of the solidifying liquid; R is a particle diameter of the main capsule particle; and N is a generation number of the main capsule particle.

12. The method for simultaneously producing two kinds of seamless capsules according to any one of claims 9 to 11, wherein the main capsule particle and the sub capsule particle are separated from each other, and then dried, respectively.
